(19) Europäisches Patentamt
European Patent Office
Office européen des brevets

(11) EP 0 728 464 B1

# (12) FASCICULE DE BREVET EUROPEEN

(45) Date de publication et mention de la délivrance du brevet:
**30.07.1997 Bulletin 1997/31**

(51) Int. Cl.$^{6}$: **A61K 7/13**

(21) Numéro de dépôt: **96400183.8**

(22) Date de dépôt: **25.01.1996**

(54) **Composition de teinture d'oxydation des fibres kératiniques comprenant un dérivé de diaminopyrazole et un coupleur hétérocyclique et procédé de teinture**

Oxidationshaarfärbemittel, enthaltend ein Diaminopyrazolderivat und einen heterocyclischen Kuppler und Färbeverfahren

Composition for dyeing keratinous fibers comprising a diaminopyrazole derivative and a heterocyclic coupleur and dyeing process

(84) Etats contractants désignés:
**DE ES FR GB IT**

(30) Priorité: **27.02.1995 FR 9502271**

(43) Date de publication de la demande:
**28.08.1996 Bulletin 1996/35**

(73) Titulaire: **L'OREAL**
**75008 Paris (FR)**

(72) Inventeurs:
- **Maubru, Mireille**
  **F-78400 Chatou (FR)**
- **Audousset, Marie-Pascale**
  **F-92600 Asnières (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 375 977**
**EP-A- 0 692 245**
**WO-A-94/08970**
**DE-A- 4 133 957**
**GB-A- 2 180 215**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.11/3.4

EP 0 728 464 B1

## Description

La présente invention a pour objet une composition pour la teinture d'oxydation des fibres kératiniques, en particulier des fibres kératiniques humaines telles que les cheveux, comprenant au moins un dérivé de diaminopyrazole à titre de base d'oxydation, en association avec au moins un coupleur hétérocyclique convenablement sélectionné, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des dérivés indoliques et en particulier le 4-hydroxyindole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet allemand DE 3 843 892 et DE 4 133 957, des compositions pour la teinture d'oxydation des fibres kératiniques comprenant un dérivé de diaminopyrazole à titre de base d'oxydation, en association avec un coupleur qui peut être choisi parmi différents type de coupleurs comme la résorcine, la 4-chloro résorcine, des métaaminophénols, des métaphénylènediamines, l'$\alpha$-naphtol, des coupleurs de type hétérocyclique tels que le 4-hydroxyindole, le 4-hydroxy 1,2-méthylènedioxybenzène, la 3-amino 5-hydroxy 2,6-diméthoxypyridine ou la 3,5-diamino 2,6-diméthoxypyridine, etc... De telles compositions ne sont cependant pas entièrement satisfaisantes notamment du point de vue de leur ténacité, et en particulier du point de vue de la tenue des colorations obtenues vis à vis de la transpiration.

Or, la demanderesse vient maintenant de découvrir qu'il est possible d'obtenir de nouvelles teintures peu sélectives et particulièrement résistantes, notamment à la transpiration, capables d'engendrer des colorations intenses, en associant au moins un dérivé de diaminopyrazole de formule (I) ci-après définie et au moins un coupleur hétérocyclique convenablement sélectionné.

Cette découverte est à la base de la présente invention.

L'invention a donc pour objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les dérivés de diaminopyrazole de formule (I) suivante et leurs sels d'addition avec un acide :

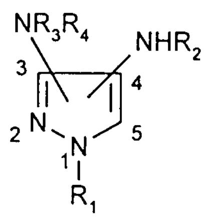

(I)

dans laquelle :

$R_1$ représente un atome d'hydrogène, un radical alkyle en $C_1$-$C_6$, hydroxyalkyle en $C_2$-$C_4$, benzyle, phényle, benzyle substitué par un atome d'halogène ou par un groupe alkyle en $C_1$-$C_4$, ou alcoxy en $C_1$-$C_4$, ou forme avec l'atome d'azote du groupement $NR_3R_4$ en position 5 un hétérocycle héxahydropyridazinique ou tétrahydropyrazolique éventuellement monosubstitué par un groupe alkyle en $C_1$-$C_4$ ;

$R_2$ et $R_3$, identiques ou différents, représentent un atome d'hydrogène, un radical alkyle en $C_1$-$C_4$, hydroxyalkyle en $C_2$-$C_4$, benzyle ou phényle ;

$R_4$ représente un atome d'hydrogène, un radical alkyle en $C_1$-$C_6$ ou hydroxyalkyle en $C_2$-$C_4$ ; sous réserve que $R_2$ représente un atome d'hydrogène lorsque $R_1$ représente un radical benzyle substitué ou forme un hétérocycle avec l'atome d'azote du groupement $NR_3R_4$ en position 5 ;

- au moins un coupleur hétérocyclique choisi parmi :

(i) les dérivés indoliques de formule (II) suivante et leurs sels d'addition avec un acide :

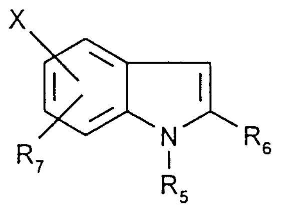

(II)

dans laquelle :

$R_5$ et $R_6$, identiques ou différents, représentent un atome d'hydrogène ou un radical alkyle en $C_1$-$C_4$ ;
$R_7$ représente un atome d'hydrogène, un radical alkyle en $C_1$-$C_4$ ou hydroxyle ;
X représente un radical hydroxyle ou $NHR_8$ dans lequel $R_8$ représente un atome d'hydrogène, un radical alkyle en $C_1$-$C_4$ ou hydroxyalkle en $C_1$-$C_4$ ;

sous réserve que :

- lorsque $R_7$ désigne hydroxyle, alors il occupe la position 6 et alors X désigne hydroxyle et occupe la position 5 et $R_5$ et $R_6$ représentent un atome d'hydrogène,
- lorsque que X désigne hydroxyle et occupe la position 4, alors au moins un des radicaux $R_5$, $R_6$ et $R_7$ est différent d'un atome d'hydrogène,
- lorsque X est en position 5, alors au moins un des radicaux $R_5$, $R_6$ et $R_7$ est différent d'un atome d'hydrogène ;

(ii) les dérivés de benzimidazole de formule (III) suivante et leurs sels d'addition avec un acide :

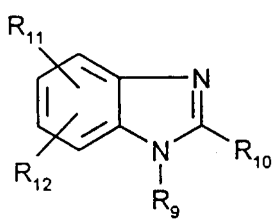

(III)

dans laquelle :

$R_9$ représente un atome d'hydrogène ou un radical alkyle en $C_1$-$C_4$,
$R_{10}$ représente un atome d'hydrogène, un radical alkyle en $C_1$-$C_4$ ou phényle,
$R_{11}$ représente un radical hydroxyle, amino ou méthoxy,
$R_{12}$ représente un atome d'hydrogène, un radical hydroxyle, méthoxy ou alkyle en $C_1$-$C_4$,

sous réserve que :

- lorsque $R_{11}$ désigne un radical amino, alors il occupe la position 4,
- lorsque $R_{11}$ occupe la position 4, alors $R_{12}$ occupe la position 7,
- lorsque $R_{11}$ occupe la position 5, alors $R_{12}$ occupe la position 6,

(iii) les dérivés de benzomorpholine de formule (IV) suivante et leurs sels d'addition avec un acide :

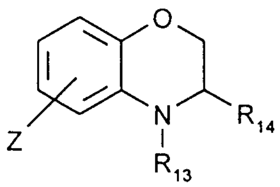

(IV)

dans laquelle :

$R_{13}$ et $R_{14}$, identiques ou différents, représentent un atome d'hydrogène ou un radical alkyle en $C_1$-$C_4$,
Z représente un radical hydroxyle ou amino ;

(iv) le 3-(3',5'-diamino 2'-pyridyloxy) 2-hydroxy propanol, les dérivés de pyridine de formule (V) suivante et leurs sels d'addition avec un acide :

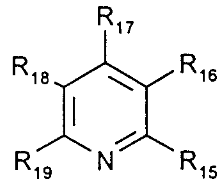

(V)

dans laquelle :

$R_{15}$ représente un atome d'hydrogène, un radical hydroxyle, amino ou $-OCH_2CH_2COCH_2CH_2OH$,
$R_{16}$ et $R_{18}$, identiques ou différents, représentent un atome d'hydrogène, un radical hydroxyle, amino ou alkyle en $C_1$-$C_4$,
$R_{17}$ représente un atome d'hydrogène ou un radical alkyle en $C_1$-$C_4$,
$R_{19}$ représente un atome d'hydrogène, un radical hydroxyle ou amino, étant entendu que ces composés de formule (V) ne comportent pas plus de deux groupements amino (substitués ou non substitués) ou pas plus de deux groupements hydroxyle ou pas plus de un groupement amino et un groupement hydroxyle par molécule, ces groupements amino et/ou hydroxyle étant obligatoirement en position méta l'un par rapport à l'autre ;

(v) les dérivés d'indoline de formule (VI) suivante et leurs sels d'addition avec un acide :

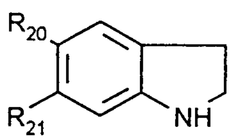

(VI)

dans laquelle :

$R_{20}$ désigne un atome d'hydrogène ou un radical hydroxyle,
$R_{21}$ désigne un radical hydroxyle ou amino ;

(vi) les dérivés de quinoline de formule (VII) suivante et leurs sels d'addition avec un acide :

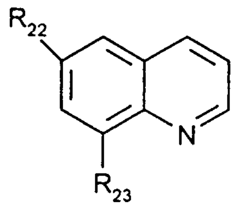

(VII)

dans laquelle :

$R_{22}$ désigne un radical hydroxyle ou alcoxy en $C_1$-$C_4$,
$R_{23}$ désigne un atome d'hydrogène ou radical amino ;

(vii) les dérivés de sésamol de formule (VIII) suivante et leurs sels d'addition avec un acide :

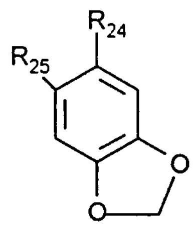

(VIII)

dans laquelle :

$R_{24}$ désigne un radical hydroxyle ou amino,
$R_{25}$ désigne un atome d'halogène ou un radical alcoxy en $C_1$-$C_4$,

sous réserve que :

- lorsque $R_{24}$ désigne un radical amino, alors $R_{25}$ désigne un radical alcoxy en $C_1$-$C_4$,
- lorsque $R_{24}$ désigne un radical hydroxyle, alors $R_{25}$ désigne un atome d'halogène ; étant entendu que lorsque ladite composition comprend un composé de formule (I) qui serait le 4,5-diamino 1-méthyl pyrazole, alors elle ne contient pas de dérivé indolique de formule (II) qui serait le 5,6-dihydroxyindole.

Les colorations obtenues avec la composition de teinture d'oxydation conforme à l'invention, présentent une bonne puissance tinctoriale et d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables, notamment en ce qui concerne la résistance des colorations obtenues vis à vis de la transpiration.

L'invention a également pour objet un procédé de teinture d'oxydation des fibres kératiniques mettant en oeuvre cette composition.

Les sels d'addition avec un acide utilisables dans le cadre des compositions tinctoriales de l'invention sont notamment choisis parmi les chlorhydrates, les bromhydrates, les sulfates et les tartrates.

Parmi les dérivés de diaminopyrazole de formule (I) ci-dessus, on peut plus particulièrement citer le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-benzyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine, et leurs sels d'addition avec un acide.

Parmi les dérivés indoliques de formule (II) ci-dessus, on peut plus particulièrement citer le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, et leurs sels d'addition avec un acide.

Parmi les dérivés de benzimidazole de formule (III) ci-dessus, on peut plus particulièrement citer le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

Parmi les dérivés de benzomorpholine de formule (IV) ci-dessus, on peut plus particulièrement citer la 6-hydroxy benzomorpholine, la N-méthyl 6-hydroxy benzomorpholine, la 6-amino benzomorpholine, et leurs sels d'addition avec un acide.

Parmi les dérivés de pyridine de formule (V) ci-dessus, on peut plus particulièrement citer la 2,6-dihydroxy 4-méthyl pyridine, la 2,6-dihydroxy 3,4-diméthyl pyridine, la 2,6-diamino pyridine, le 3-oxo 5-(3',5'-diamino 2'-pyridiloxy) pentanol, et leurs sels d'addition avec un acide.

Parmi les dérivés d'indoline de formule (VI) ci-dessus, on peut citer la 6-hydroxy indoline, la 6-amino indoline, la 5,6-dihydroxy indoline, et leurs sels d'addition avec un acide.

Parmi les dérivés de quinoline de formule (VII) ci-dessus, on peut plus particulièrement citer la 6-hydroxy quinoline, la 8-amino 6-méthoxy quinoline, et leurs sels d'addition avec un acide.

Parmi les dérivés de sésamol de formule (VIII) ci-dessus, on peut plus particulièrement citer le 2-bromo 4,5-méthylènedioxy phénol, la 2-méthoxy 4,5-méthylènedioxy aniline, et leurs sels d'addition avec un acide.

Le ou les dérivés de diaminopyrazole de formule (I) conformes à l'invention représentent de préférence de 0,0005 à 12 % en poids environ du poids total de la composition tinctoriale, et encore plus préférentiellement de 0,005 à 6 % en poids environ de ce poids.

Le ou les coupleurs hétérocycliques conformes à l'invention représentent de préférence de 0,0001 à 10 % en poids environ du poids total de la composition tinctoriale, et encore plus préférentiellement de 0,005 à 5 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IX) suivante :

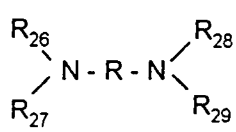

(IX)

dans laquelle R est un reste propylène éventuellement substitué par un groupement hydroxyle ou un radical alkyle en $C_1$-$C_4$; $R_{26}$, $R_{27}$, $R_{28}$ et $R_{29}$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, d'autres bases d'oxydation et/ou d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de

conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à l'association binaire conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLES

Les compositions tinctoriales A-F suivantes ont été préparées (teneurs en grammes) :

| COMPOSITION | A (*) | B | C | D | E | F |
|---|---|---|---|---|---|---|
| Dichlorhydrate de 4,5-diamino pyrazole | 0,513 | 0,513 | 0,513 | 0,513 | 0,513 | 0,513 |
| 4-hydroxy indole | 0,399 | | | | | |
| 4-hydroxy 1-N-méthyl indole | | 0,442 | | | | |
| Bromhydrate de 7-amino indole | | | 0,639 | | | |
| 6-hydroxy indole | | | | 0,399 | | |
| Bromhydrate de 4-hydroxy benzimidazole | | | | | 0,645 | |
| Chlorhydrate de 6-hydroxy indoline | | | | | | 0,515 |
| Support de teinture commun (**) | (**) | (**) | (**) | (**) | (**) | (**) |
| Eau déminéralisée q.s.p. | 100 g | 100 g | 100 g | 100 g | 100 g | 100 g |

(*) : Composition tinctoriale ne faisant pas partie de l'invention

(**) : Support de teinture commun :

- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g

| | | |
|---|---|---|
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, on a mélangé chaque composition tinctoriale avec une quantité égale en poids d'une composition oxydante constituée par une solution d'eau oxygénée à 20 volumes (6 % en poids).

Chaque mélange obtenu a été appliqué pendant 30 minutes sur des mèches de cheveux gris permanentés à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Les mèches de cheveux ainsi teintes ont ensuite été soumises à un test de résistance à la transpiration.

Pour ce faire, les mèches de cheveux ont été immergées dans un cristallisoir recouvert d'un verre de montre et renfermant une solution de sueur synthétique de composition suivante :

| | |
|---|---|
| - NaCl | 1 g |
| - Hydrogénophosphate de potassium | 0,1 g |
| - Histidine | 0,025 g |
| - Acide lactique q.s. | pH 3,2 |
| - Eau distillée q.s.p. | 100 g |

On a laissé séjourner les mèches de cheveux teints dans cette solution de sueur synthétique pendant 48 heures à 37° C. Les mèches ont ensuite été rincées puis séchées.

La couleur des mèches a été ensuite évaluée à nouveau dans le système MUNSELL au moyen d'un colorimètre CM 2002 MINOLTA de façon à déterminer la dégradation des colorations après ce test de résistance à la transpiration.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur entre deux mèches (ici, avant et après le traitement simulant une transpiration) est calculée en appliquant la formule de NICKERSON :

$$\Delta E = 0{,}4\ Co\Delta H + 6\Delta V + 3\ \Delta C,$$

telle que décrite par exemple dans "Couleur, Industrie et Technique" ; pages 14-17 ; vol. n° 5; 1978.

Dans cette formule, **ΔE** représente la différence de couleur entre deux mèches, ΔH, ΔV et ΔC représentent la variation en valeur absolue des paramètres **H**, **V** et **C** et **Co** représente la pureté de la mèche par rapport à laquelle on désire évaluer la différence de couleur.

Les résultats sont donnés dans le tableau ci-après :

| COMPOSITION | Couleur des cheveux avant le test | Couleur des cheveux après le test | Dégradation de la couleur | | | |
|---|---|---|---|---|---|---|
| | | | ΔH | ΔV | ΔC | **ΔE** |
| | | | | | | |
| **A(*)** | 6,9 RP 2,8/6,3 | 8,8 RP 2,9/3,4 | 1,9 | 0,1 | 2,9 | **14,1** |
| **B** | 6,1 RP 3,1/6,1 | 8,3 RP 3,4/5,2 | 2,2 | 0,3 | 0,9 | **9,9** |
| **C** | 1,1 B 3,4/0,2 | 9,0 YR 3,9/1,4 | 42,2 | 0,5 | 1,2 | **10,0** |
| **D** | 4,3 YR 4,4/3,9 | 7,3 YR 4,8/2,9 | 3,0 | 0,4 | 1,0 | **10,1** |
| **E** | 8,4 RP 2,8/6,8 | 8,3 RP 3,3/5,1 | 0,1 | 0,5 | 1,7 | **8,4** |
| **F** | 8,0 R 3,6/1,2 | 2,3 YR 3,5/1,6 | 4,3 | 0,1 | 0,4 | **3,9** |

(*) : Composition ne faisant pas partie de l'invention

Ces résultats montrent que les compositions B à F conformes à l'invention conduisent à une coloration résistant beaucoup mieux à la transpiration que la composition A ne faisant pas partie de l'invention.

**Revendications**

**1.** Composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les dérivés de diaminopyrazole de formule (I) suivante et leurs sels d'addition avec un acide :

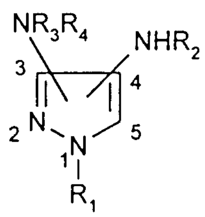

(I)

dans laquelle :

$R_1$ représente un atome d'hydrogène, un radical alkyle en $C_1$-$C_6$, hydroxyalkyle en $C_2$-$C_4$, benzyle, phényle, benzyle substitué par un atome d'halogène ou par un groupe alkyle en $C_1$-$C_4$, ou alcoxy en $C_1$-$C_4$, ou forme avec l'atome d'azote du groupement $NR_3R_4$ en position 5 un hétérocycle héxahydropyridazinique ou tétrahydropyrazolique éventuellement monosubstitué par un groupe alkyle en $C_1$-$C_4$ ;
$R_2$ et $R_3$, identiques ou différents, représentent un atome d'hydrogène, un radical alkyle en $C_1$-$C_4$, hydroxyalkyle en $C_2$-$C_4$, benzyle ou phényle ;
$R_4$ représente un atome d'hydrogène, un radical alkyle en $C_1$-$C_6$ ou hydroxyalkyle en $C_2$-$C_4$ ; sous réserve que $R_2$ représente un atome d'hydrogène lorsque $R_1$ représente un radical benzyle substitué ou forme un

hétérocycle avec l'atome d'azote du groupement $NR_3R_4$ en position 5 ;

- au moins un coupleur hétérocyclique choisi parmi :

(i) les dérivés indoliques de formule (II) suivante et leurs sels d'addition avec un acide :

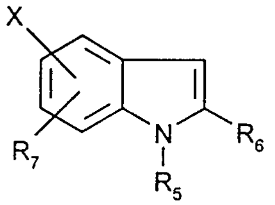

(II)

dans laquelle :

$R_5$ et $R_6$, identiques ou différents, représentent un atome d'hydrogène ou un radical alkyle en $C_1$-$C_4$;
$R_7$ représente un atome d'hydrogène, un radical alkyle en $C_1$-$C_4$ ou hydroxyle;
X représente un radical hydroxyle ou $NHR_8$ dans lequel $R_8$ représente un atome d'hydrogène, un radical alkyle en $C_1$-$C_4$ ou hydroxyalkle en $C_1$-$C_4$;

sous réserve que :

- lorsque $R_7$ désigne hydroxyle, alors il occupe la position 6 et alors X désigne hydroxyle et occupe la position 5 et $R_5$ et $R_6$ représentent un atome d'hydrogène,
- lorsque que X désigne hydroxyle et occupe la position 4, alors au moins un des radicaux $R_5$, $R_6$ et $R_7$ est différent d'un atome d'hydrogène,
- lorsque X est en position 5, alors au moins un des radicaux $R_5$, $R_6$ et $R_7$ est différent d'un atome d'hydrogène ;

(ii) les dérivés de benzimidazole de formule (II) suivante et leurs sels d'addition avec un acide :

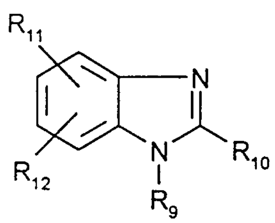

(III)

dans laquelle :

$R_9$ représente un atome d'hydrogène ou un radical alkyle en $C_1$-$C_4$,
$R_{10}$ représente un atome d'hydrogène, un radical alkyle en $C_1$-$C_4$ ou phényle,
$R_{11}$ représente un radical hydroxyle, amino ou méthoxy,
$R_{12}$ représente un atome d'hydrogène, un radical hydroxyle, méthoxy ou alkyle en $C_1$-$C_4$;

sous réserve que :

- lorsque $R_{11}$ désigne un radical amino, alors il occupe la position 4,
- lorsque $R_{11}$ occupe la position 4, alors $R_{12}$ occupe la position 7,
- lorsque $R_{11}$ occupe la position 5, alors $R_{12}$ occupe la position 6;

(iii) les dérivés de benzomorpholine de formule (IV) suivante et leurs sels d'addition avec un acide :

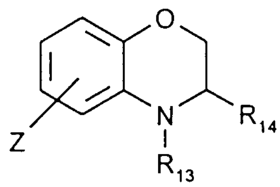

(IV)

dans laquelle :

$R_{13}$ et $R_{14}$, identiques ou différents, représentent un atome d'hydrogène ou un radical alkyle en $C_1$-$C_4$,
Z représente un radical hydroxyle ou amino ;

(iv) le 3-(3',5'-diamino 2'-pyridyloxy) 2-hydroxy propanol, les dérivés de pyridine de formule (V) suivante et leurs sels d'addition avec un acide :

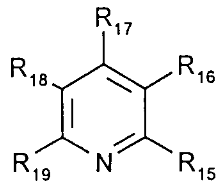

(V)

dans laquelle :

$R_{15}$ représente un atome d'hydrogéne, un radical hydroxyle, amino ou $-OCH_2CH_2COCH_2CH_2OH$,
$R_{16}$ et $R_{18}$, identiques ou différents, représentent un atome d'hydrogène, un radical hydroxyle, amino ou alkyle en $C_1$-$C_4$,
$R_{17}$ représente un atome d'hydrogène ou un radical alkyle en $C_1$-$C_4$,
$R_{19}$ représente un atome d'hydrogène, un radical hydroxyle ou amino, étant entendu que ces composés de formule (V) ne comportent pas plus de deux groupements amino (substitués ou non substitués) ou pas plus de deux groupements hydroxyle ou pas plus de un groupement amino et un groupement hydroxyle par molécule, ces groupements amino et/ou hydroxyle étant obligatoirement en position méta l'un par rapport à l'autre ;

(v) les dérivés d'indoline de formule (VI) suivante et leurs sels d'addition avec un acide :

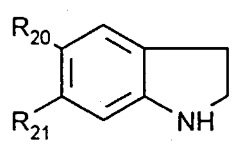

(VI)

dans laquelle :

$R_{20}$ désigne un atome d'hydrogène ou un radical hydroxyle,
$R_{21}$ désigne un radical hydroxyle ou amino ;

(vi) les dérivés de quinoline de formule (VII) suivante et leurs sels d'addition avec un acide :

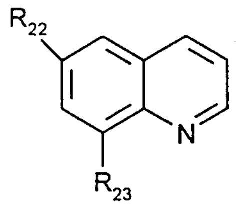

(VII)

dans laquelle :

$R_{22}$ désigne un radical hydroxyle ou alcoxy en $C_1$-$C_4$,
$R_{23}$ désigne un atome d'hydrogène ou radical amino ;

(vii) les dérivés de sésamol de formule (VIII) suivante et leurs sels d'addition avec un acide :

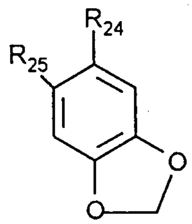

(VIII)

dans laquelle :

$R_{24}$ désigne un radical hydroxyle ou amino,
$R_{25}$ désigne un atome d'halogène ou un radical alcoxy en $C_1$-$C_4$,

sous réserve que :

- lorsque $R_{24}$ désigne un radical amino, alors $R_{25}$ désigne un radical alcoxy en $C_1$-$C_4$,
- lorsque $R_{24}$ désigne un radical hydroxyle, alors $R_{25}$ désigne un atome d'halogène ;

étant entendu que lorsque ladite composition comprend un composé de formule (I) qui serait le 4,5-diamino 1-méthyl pyrazole, alors elle ne contient pas de dérivé indolique de formule (II) qui serait le 5,6-dihydroxyindole.

**2.** Composition selon la revendication 1, caractérisée par le fait que les sels d'addition avec un acide sont choisis parmi les chlorhydrates, les bromhydrates, les sulfates et les tartrates.

**3.** Composition selon la revendication 1 ou 2, caractérisée par le fait que les dérivés de diaminopyrazole de formule (I) sont choisis parmi le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-benzyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine, et leurs sels d'addition avec un acide.

**4.** Composition selon la revendication 1 ou 2, caractérisée par le fait que les dérivés indoliques de formule (II) sont choisis parmi le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, et leurs sels d'addition avec un acide.

**5.** Composition selon la revendication 1 ou 2, caractérisée par le fait que les dérivés de benzimidazole de formule (III) sont choisis parmi le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy

1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

6. Composition selon la revendication 1 ou 2, caractérisée par le fait que les dérivés de benzomorpholine de formule (IV) sont choisis parmi la 6-hydroxy benzomorpholine, la N-méthyl 6-hydroxy benzomorpholine, la 6-amino benzomorpholine, et leurs sels d'addition avec un acide.

7. Composition selon la revendication 1 ou 2, caractérisée par le fait que les dérivés de pyridine de formule (V) sont choisis parmi la 2,6-dihydroxy 4-méthyl pyridine, la 2,6-dihydroxy 3,4-diméthyl pyridine, la 2,6-diamino pyridine, le 3-oxo 5-(3',5'-diamino 2'-pyridiloxy) pentanol, et leurs sels d'addition avec un acide.

8. Composition selon la revendication 1 ou 2, caractérisée par le fait que les dérivés d'indoline de formule (VI) sont choisis parmi la 6-hydroxy indoline, la 6-amino indoline, la 5,6-dihydroxy indoline, et leurs sels d'addition avec un acide.

9. Composition selon la revendication 1 ou 2, caractérisée par le fait que les dérivés de quinoline de formule (VII) sont choisis parmi la 6-hydroxy quinoline, la 8-amino 6-méthoxy quinoline, et leurs sels d'addition avec un acide.

10. Composition selon la revendication 1 ou 2, caractérisée par le fait que les dérivés de sésamol de formule (VIII) sont choisis parmi le 2-bromo 4,5-méthylènedioxy phénol, la 2-méthoxy 4,5-méthylénedioxy aniline, et leurs sels d'addition avec un acide.

11. Composition selon l'une quelconque des revendications 1 à 10, caractérisée par le fait que le ou les dérivés de diaminopyrazole de formule (I) représentent de 0,0005 à 12 % en poids du poids total de la composition tinctoriale.

12. Composition selon la revendication 11, caractérisée par le fait que le ou les dérivés de diaminopyrazole de formule (I) représentent de 0,005 à 6 % en poids du poids total de la composition tinctoriale.

13. Composition selon l'une quelconque des revendications 1 à 12, caractérisée par le fait que le ou les coupleurs hétérocycliques représentent de 0,0001 à 10% en poids du poids total de la composition tinctoriale.

14. Composition selon la revendication 13, caractérisée par le fait que le ou les coupleurs hétérocycliques représentent de 0,005 à 5 % en poids du poids total de la composition tinctoriale.

15. Composition selon l'une quelconque des revendications 1 à 14, caractérisée par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques et leurs mélanges.

16. Composition selon l'une quelconque des revendications 1 à 15, caractérisée par le fait qu'elle présente un pH compris entre 3 et 12.

17. Procédé de teinture des fibres kératiniques et en particulier des fibres kératiniques humaines telles que les cheveux, caractérisé par le fait que l'on applique sur ces fibres une composition tinctoriale telle que définie à l'une quelconque des revendications 1 à 16 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

18. Procédé selon la revendication 17, caractérisé par le fait que l'agent oxydant est choisi parmi le peroxyde d'hydrogène, le peroxyde d'urée, les bromates de métaux alcalins, les persels tels que les perborates et persulfates.

19. Dispositif à plusieurs compartiments, ou "kit" de teinture à plusieurs compartiments, dont un premier compartiment renferme une composition tinctoriale telle que définie à l'une quelconque des revendications 1 à 16 et un second compartiment renferme une composition oxydante.

## Claims

1. Composition for the oxidation dyeing of keratin fibres, and in particular human keratin fibres such as the hair, characterized in that it comprises, in a medium which is suitable for dyeing:

- at least one oxidation base chosen from the diaminopyrazole derivatives of following formula (I), and the addition salts thereof with an acid:

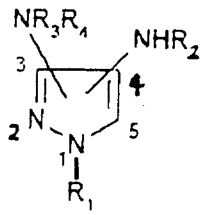

(I)

in which:

$R_1$ represents a hydrogen atom, a $C_1$-$C_6$ alkyl, $C_2$-$C_4$ hydroxyalkyl, benzyl or phenyl radical, a benzyl radical substituted with a halogen atom or with a $C_1$-$C_4$ alkyl or $C_1$-$C_4$ alkoxy group, or forms, with the nitrogen atom of the $NR_3R_4$ group in the 5-position, a hexahydropyridazine or tetrahydroimidazole heterocycle optionally mono-substituted with a $C_1$-$C_4$ alkyl group;
$R_2$ and $R_3$, which may be identical or different, represent a hydrogen atom or a $C_1$-$C_4$ alkyl, $C_2$-$C_4$ hydroxyalkyl, benzyl or phenyl radical;
$R_4$ represents a hydrogen atom or a $C_1$-$C_6$ alkyl or $C_2$-$C_4$ hydroxyalkyl radical; with the proviso that $R_2$ represents a hydrogen atom when $R_1$ represents a substituted benzyl radical, or forms a heterocycle with the nitrogen atom of the $NR_3R_4$ group in the 5-position;

- at least one heterocyclic coupler chosen from:

(i) indole derivatives of following formula (II), and the addition salts thereof with an acid:

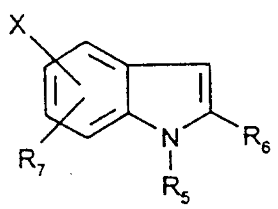

(II)

in which:

$R_5$ and $R_6$, which may be identical or different, represent a hydrogen atom or a $C_1$-$C_4$ alkyl radical;
$R_7$ represents a hydrogen atom or a $C_1$-$C_4$ alkyl or hydroxyl radical;
X represents a hydroxyl radical or a radical $NHR_8$ in which $R_8$ represents a hydrogen atom or a $C_1$-$C_4$ alkyl or $C_1$-$C_4$ hydroxyalkyl radical;

with the proviso that:

- when $R_7$ denotes hydroxyl, it then occupies the 6-position and X then denotes hydroxyl and occupies the 5-position and $R_5$ and $R_6$ represent a hydrogen atom,
- when X denotes hydroxyl and occupies the 4-position, then at least one of the radicals $R_5$, $R_6$ and $R_7$ is other than a hydrogen atom,
- when X is in the 5-position, then at least one of the radicals $R_5$, $R_6$ and $R_7$ is other than a hydrogen atom;

(ii) the benzimidazole derivatives of following formula (III), and the addition salts thereof with an acid:

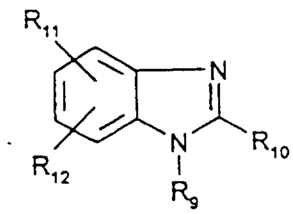

(III)

in which:

$R_9$ represents a hydrogen atom or a $C_1$-$C_4$ alkyl radical,
$R_{10}$ represents a hydrogen atom or a $C_1$-$C_4$ alkyl or phenyl radical,
$R_{11}$ represents a hydroxyl, amino or methoxy radical,
$R_{12}$ represents a hydrogen atom or a hydroxyl, methoxy or $C_1$-$C_4$ alkyl radical;

with the proviso that:

- when $R_{11}$ denotes an amino radical, it then occupies the 4-position,
- when $R_{11}$ occupies the 4-position, $R_{12}$ then occupies the 7-position,
- when $R_{11}$ occupies the 5-position, $R_{12}$ then occupies the 6-position;

(iii) the benzomorpholine derivatives of following formula (IV), and the addition salts thereof with an acid:

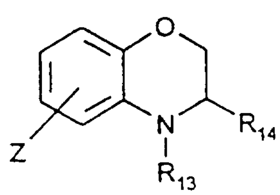

(IV)

in which:

$R_{13}$ and $R_{14}$, which may be identical or different, represent a hydrogen atom or a $C_1$-$C_4$ alkyl radical,
Z represents a hydroxyl or amino radical;

(iv) 3-(3',5'-diamino-2'-pridyloxy)-2-hydroxypropanol, the pyridine derivatives of following formula (V), and the addition salts thereof with an acid:

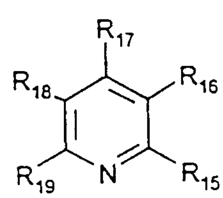

(V)

in which:

$R_{15}$ represents a hydrogen atom or a hydroxyl, amino or $-OCH_2CH_2COCH_2CH_2OH$ radical,
$R_{16}$ and $R_{18}$, which may be identical or different, represent a hydrogen atom or a hydroxyl, amino or $C_1$-$C_4$ alkyl radical,
$R_{17}$ represents a hydrogen atom or a $C_1$-$C_4$ alkyl radical,
$R_{19}$ represents a hydrogen atom or a hydroxyl or amino radical, it being understood that these compounds of formula (V) do not contain more than two amino groups (substituted or unsubstituted) or not more than two hydroxyl groups or not more than one amino group and one hydroxyl group per molecule, these amino and/or hydroxyl groups necessarily being in a meta position relative to each other;

(v) indoline derivatives of following formula (VI), and the addition salts thereof with an acid:

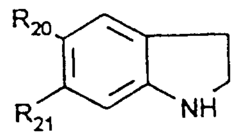

(VI)

in which:

$R_{20}$ denotes a hydrogen atom or a hydroxyl radical,
$R_{21}$ denotes a hydroxyl or amino radical;

(vi) the quinoline derivatives of following formula (VII), and the addition salts thereof with an acid:

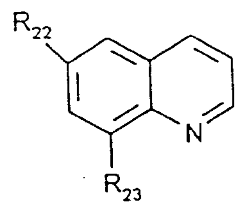

(VII)

in which:

$R_{22}$ denotes a hydroxyl or $C_1$-$C_4$ alkoxy radical,
$R_{23}$ denotes a hydrogen atom or amino radical;

(vii) the sesamol derivatives of following formula (VIII), and the addition salts thereof with an acid:

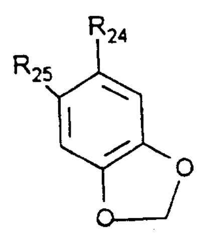

(VIII)

in which:

$R_{24}$ denotes a hydroxyl or amino radical,
$R_{25}$ denotes a halogen atom or a $C_1$-$C_4$ alkoxy radical,

with the proviso that:

- when $R_{24}$ denotes an amino radical, $R_{25}$ then denotes a $C_1$-$C_4$ alkoxy radical,
- when $R_{24}$ denotes a hydroxyl radical, $R_{25}$ then denotes a halogen atom;

it being understood that when the said composition comprises a compound of formula (I) which is 4,5-diamino-1-methylpyrazole, it then does not contain any indole derivative of formula (II) which is 5,6-dihydroxyindole.

**2.** Composition according to Claim 1, characterized in that the addition salts with an acid are chosen from the hydrochlorides, hydrobromides, sulphates and tartrates.

**3.** Composition according to Claim 1 or 2, characterized in that the diaminopyrazole derivatives of formula (I) are chosen from 4,5-diamino-1-(4'-methoxybenzyl)pyrazole, 4,5-diamino-1-(4'-methylbenzyl)pyrazole, 4,5-diamino-1-(4'-chlorobenzyl)pyrazole, 4,5-diamino-1-(3'-methoxybenzyl)pyrazole, 4-amino-1-(4'-methoxybenzyl)-5-methylaminopyrazole, 4-amino-5-(β-hydroxyethyl)amino-1-(4'-methoxybenzyl)pyrazole, 4-amino-5-(β-hydroxyethyl)amino-1-methylpyrazole, 4-amino-(3) 5-methylaminopyrazole, 3(5),4-diaminopyrazole, 4,5-diamino-1-methylpyrazole, 4,5-diamino-1-benzylpyrazole, 3-amino-4,5,7,8-tetrahydropyrazolo[1,5-a]pyrimidine, 7-amino-2,3-dihydro-1H-imidazo[1,2-b]pyrazole and 3-amino-8-methyl-4,5,7,8-tetrahydropyrazolo[1,5-a]pyrimidine, and the addition salts thereof with an acid.

**4.** Composition according to Claim 1 or 2, characterized in that the indole derivatives of formula (II) are chosen from 6-hydroxyindole, 7-aminoindole, 6-aminoindole, 7-hydroxyindole, 7-ethyl-6-(β-hydroxyethyl)aminoindole, 4-aminoindole, 6-hydroxy-1-methylindole, 5,6-dihydroxyindole, 4-hydroxy-1-N-methylindole, 4-hydroxy-2-methylindole and 4-hydroxy-5-methylindole, and the addition salts thereof with an acid.

**5.** Composition according to Claim 1 or 2, characterized in that the benzimidazole derivatives of formula (III) are chosen from 4-hydroxybenzimidazole, 4-aminobenzimidazole, 4-hydroxy-7-methylbenzimidazole, 4-hydroxy-2-methylbenzimidazole, 1-butyl-4-hydroxybenzimidazole, 4-amino-2-methylbenzimidazole, 5,6-dihydroxybenzimidazole, 5-hydroxy-6-methoxybenzimidazole, 4,7-dihydroxybenzimidazole, 4,7-dihydroxy-1-methylbenzimidazole, 4,7-dimethoxybenzimidazole, 5,6-dihydroxy1-methylbenzimidazole, 5,6-dihydroxy-2-methylbenzimidazole and 5,6-dimethoxybenzimidazole, and the addition salts thereof with an acid.

**6.** Composition according to Claim 1 or 2, characterized in that the benzomorpholine derivatives of formula (IV) are chosen from 6-hydroxybenzomorpholine, N-methyl-6-hydroxybenzomorpholine and 6-aminobenzomorpholine, and the addition salts thereof with an acid.

**7.** Composition according to Claim 1 or 2, characterized in that the pyridine derivatives of formula (V) are chosen from 2,6-dihydroxy-4-methylpyridine, 2,6-dihydroxy-3,4-dimethylpyridine, 2,6-diaminopyridine and 3-oxo-5-(3',5'-diamino-2'-pyridyloxy)pentanol, and the addition salts thereof with an acid.

**8.** Composition according to Claim 1 or 2, characterized in that the indoline derivatives of formula (VI) are chosen from 6-hydroxyindoline, 6-aminoindoline and 5,6-dihydroxyindoline, and the addition salts thereof with an acid.

**9.** Composition according to Claim 1 or 2, characterized in that the quinoline derivatives of formula (VII) are chosen from 6-hydroxyquinoline and 8-amino-6-methoxyquinoline, and the addition salts thereof with an acid.

**10.** Composition according to Claim 1 or 2, characterized in that the sesamol derivatives of formula (VIII) are chosen from 2-bromo-4,5-methylenedioxyphenol and 2-methoxy-4,5-methylenedioxyaniline, and the addition salts thereof with an acid.

**11.** Composition according to any one of Claims 1 to 10, characterized in that the diaminopyrazole derivative or derivatives of formula (I) represent from 0.0005 to 12 % by weight relative to the total weight of the dye composition.

**12.** Composition according to Claim 11, characterized in that the diaminopyrazole derivative or derivatives of formula (I) represent from 0.005 to 6 % by weight relative to the total weight of the dye composition.

**13.** Composition according to any one of Claims 1 to 12, characterized in that the heterocyclic coupler or couplers represent from 0.0001 to 10 % by weight relative to the total weight of the dye composition.

**14.** Composition according to Claim 13, characterized in that the heterocyclic coupler or couplers represent from 0.005 to 5 % by weight relative to the total weight of the dye composition.

**15.** Composition according to any one of Claims 1 to 14, characterized in that the appropriate medium for the dyeing (or the support) consists of water or of a mixture of water and at least one organic solvent chosen from $C_1$-$C_4$ lower alkanols, glycerol, glycols and glycolethers, aromatic alcohols and mixtures thereof.

**16.** Composition according to any one of Claims 1 to 15, characterized in that it has a pH of between 3 and 12.

**17.** Process for dyeing keratin fibres, and in particular human keratin fibres such as the hair, characterized in that a dye composition as defined in any one of Claims 1 to 16 is applied to these fibres and in that the colour is developed at acidic, neutral or alkaline pH using an oxidizing agent which is added only at the time of use to the dye composition or which is present in an oxidizing composition that is applied simultaneously or sequentially in a separate manner.

18. Process according to Claim 17, characterized in that the oxidizing agent is chosen from hydrogen peroxide, urea peroxide, alkali metal bromates and persalts such as perborates and persulphates.

19. Multi-compartment device, or multi-compartment dyeing "kit", a first compartment of which contains a dye composition as defined in any one of Claims 1 to 16 and a second compartment of which contains an oxidizing composition.

**Patentansprüche**

1. Zusammensetzung zum oxidativen Färben von Keratinfasern und insbesondere von menschlichen Keratinfasern, wie dem Haar,
**dadurch gekennzeichnet, daß**
sie in einem zum Färben geeigneten Medium enthält:

- mindestens eine Oxidationsbase, die unter den Diaminopyrazolderivaten der folgenden Formel (I) und den Additionssalzen dieser Verbindungen mit einer Säure ausgewählt ist:

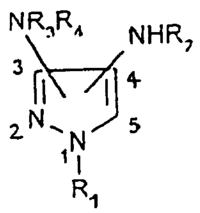

(I),

worin bedeuten:

| | |
|---|---|
| $R_1$ | ein Wasserstoffatom, $C_{1-6}$-Alkyl, $C_{2-4}$-Hydroxyalkyl, Benzyl, Phenyl, mit einem Halogenatom oder einer $C_{1-4}$-Alkylgruppe substituiertes Benzyl oder $C_{1-4}$-Alkoxy, oder $R_1$ bildet mit dem Stickstoffatom der Gruppe $NR_3R_4$ in 5-Stellung einen Hexahydropyridazin- oder Tetrahydropyrazol-Heterocyclus, der gegebenenfalls mit einer $C_{1-4}$-Gruppe einfach substituiert ist, |
| $R_2$ und $R_3$, | die gleich oder voneinander verschieden sind, Wasserstoff, $C_{1-4}$-Alkyl, $C_{2-4}$-Hydroxyalkyl, Benzyl oder Phenyl und |
| $R_4$ | Wasserstoff, $C_{1-6}$-Alkyl oder $C_{2-4}$-Hydroxyalkyl, |

mit der Maßgabe, daß $R_2$ ein Wasserstoffatom bedeutet, wenn $R_1$ eine substituierte Benzylgruppe bedeutet oder mit dem Stickstoffatom der Gruppe $NR_3R_4$ in 5-Stellung einen Heterocyclus bildet und

- mindestens einen heterocyclischen Kuppler, der ausgewählt ist unter:

(i) Indolderivaten der folgenden Formel (II) und den Additionssalzen dieser Verbindungen mit einer Säure:

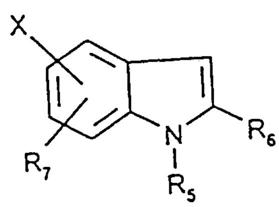

(II),

worin bedeuten:

| | |
|---|---|
| $R_5$ und $R_6$, | die identisch oder voneinander verschieden sind, Wasserstoff oder $C_{1-4}$-Alkyl; |
| $R_7$ | Wasserstoff, $C_{1-4}$-Alkyl oder Hydroxy und |
| X | Hydroxy oder $NHR_8$, worin $R_8$ ein Wasserstoffatom, $C_{1-4}$-Alkyl oder $C_{1-4}$-Hydroxyalkyl bedeutet, mit den Maßgaben, daß: |

- wenn $R_7$ Hydroxy bedeutet, $R_7$ sich in 6-Stellung befindet und X Hydroxy bedeutet und sich in 5-Stellung befindet und $R_5$ und $R_6$ ein Wasserstoffatom bedeuten,

- wenn X Hydroxy bedeutet und sich in 4-Stellung befindet, mindestens eine der Gruppen $R_5$, $R_6$ und $R_7$ von Wasserstoff verschieden ist,

- wenn X sich in 5-Stellung befindet, mindestens eine der Gruppen $R_5$, $R_6$ und $R_7$ von Wasserstoff verschieden ist;

(ii) Benzimidazolderivaten der folgenden Formel (III) und den Additionssalzen dieser Verbindungen mit einer Säure:

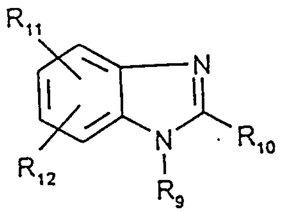

(III),

worin bedeuten:

$R_9$ Wasserstoff oder $C_{1-4}$-Alkyl,
$R_{10}$ Wasserstoff, $C_{1-4}$-Alkyl oder Phenyl,
$R_{11}$ Hydroxy, Amino oder Methoxy
und
$R_{12}$ Wasserstoff, Hydroxy, Methoxy oder $C_{1-4}$-Alkyl,

mit den Maßgaben, daß:

- wenn $R_{11}$ Amino bedeutet, $R_{11}$ sich in 4-Stellung befindet,

- wenn sich $R_{11}$ in 4-Stellung befindet, $R_{12}$ sich in 7-Stellung befindet,

- wenn sich $R_{11}$ in 5-Stellung befindet, $R_{12}$ sich in 6-Stellung befindet;

(iii) Benzomorpholinderivaten der folgenden Formel (IV) und den Additionssalzen dieser Verbindungen mit einer Säure:

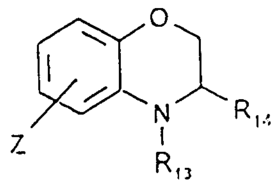

(IV),

worin bedeuten:

$R_{13}$ und $R_{14}$, die gleich oder voneinander verschieden sind, Wasserstoff oder $C_{1-4}$-Alkyl
und
Z Hydroxy oder Amino;

(iv) 3-(3',5'-Diamino-2'-pyridyloxy)-2-hydroxypropanol sowie Pyridinderivaten der folgenden Formel (V) und den Additionssalzen dieser Verbindungen mit einer Säure:

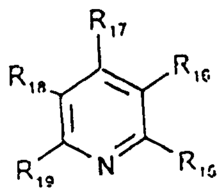

(V),

worin bedeuten:

- $R_{15}$ Wasserstoff, Hydroxy, Amino oder $OCH_2CH_2COCH_2CH_2OH$,
- $R_{16}$ und $R_{18}$, die gleich oder voneinander verschieden sind, Wasserstoff, Hydroxy, Amino oder $C_{1-4}$-Alkyl,
- $R_{17}$ Wasserstoff oder $C_{1-4}$-Alkyl und
- $R_{19}$ Wasserstoff, Hydroxy oder Amino,

mit der Maßgabe, daß die Verbindungen der Formel (V) nicht mehr als zwei Aminogruppen (substituiert oder unsubstituiert) oder nicht mehr als zwei Hydroxygruppen oder nicht mehr als eine Aminogruppe und eine Hydroxygruppe pro Molekül enthalten, wobei die Amino- und/oder Hydroxygruppen zueinander in m-Stellung stehen müssen;

(v) Indolinderivaten der folgenden Formel (VI) und den Additionssalzen dieser Verbindungen mit einer Säure:

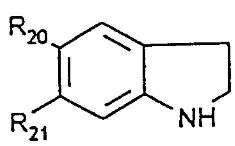

(VI),

worin bedeuten:

- $R_{20}$ Wasserstoff oder Hydroxy und
- $R_{21}$ Hydroxy oder Amino;

(vi) Chinolinderivaten der folgenden Formel (VII) und den Additionssalzen dieser Verbindungen mit einer Säure:

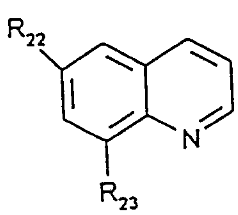

(VII),

worin bedeuten:

- $R_{22}$ Hydroxy oder $C_{1-4}$-Alkoxy und
- $R_{23}$ Wasserstoff oder Amino

sowie

(vii)Sesamolderivaten der folgenden Formel (VIII) und den Additionssalzen dieser Verbindungen mit einer Säure:

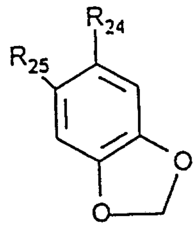

(VIII),

worin bedeuten:

$R_{24}$ Hydroxy oder Amino
und
$R_{25}$ Halogen oder $C_{1-4}$-Alkoxy,

mit den Maßgaben, daß:

- wenn $R_{24}$ Amino bedeutet, $R_{25}$ $C_{1-4}$-Alkoxy bedeutet,
- wenn $R_{24}$ Hydroxy bedeutet, $R_{25}$ Halogen bedeutet;

mit der Maßgabe, daß die Zusammensetzung kein Indolderivat der Formel (II), das 5,6-Dihydroxyindol ist, enthält, wenn sie eine Verbindung der Formel (I) enthält, die 4,5-Diamino-1-methylpyrazol ist.

**2.** Zusammensetzung nach Anspruch 1, dadurch gekennzeichnet, daß die Additionssalze mit einer Säure unter den Hydrochloriden, Hydrobromiden, Sulfaten und Tartraten ausgewählt sind.

**3.** Zusammensetzung nach Anspruch 1 oder 2, dadurch gekennzeichnet, daß die Diaminopyrazolderivate der Formel (I) ausgewählt sind unter 4,5-Diamino-1-(4'-methoxybenzyl)-pyrazol, 4,5-Diamino-1-(4'-methylbenzyl)-pyrazol, 4,5-Diamino-1-(4'-chlorbenzyl)-pyrazol, 4,5-Diamino-1-(3'-methoxybenzyl)-pyrazol, 4-Amino-1-(4'-methoxybenzyl)-5-methylaminopyrazol, 4-Amino-5-(β-hydroxyethyl)-amino-1-(4'-methoxybenzyl)-pyrazol, 4-Amino-5-(β-hydroxyethyl)-amino-1-methyl-pyrazol, 4-Amino-(3)5-methylaminopyrazol, 3(5),4-Diaminopyrazol, 4,5-Diamino-1-methylpyrazol, 4,5-Diamino-1-benzylpyrazol, 3-Amino-4,5,7,8-tetrahydro-pyrazolo[1,5-a]-pyrimidin, 7-Amino-2,3-dihydro-1H-imidazolo-[1,2-b]-pyrazol und 3-Amino-8-methyl-4,5,7,8-tetrahydropyrazolo[1,5-a]-pyrimidin sowie den Additionssalzen dieser Verbindungen mit einer Säure.

**4.** Zusammensetzung nach Anspruch 1 oder 2, dadurch gekennzeichnet, daß die Indolderivate der Formel (II) ausgewählt sind unter 6-Hydroxyindol, 7-Aminoindol, 6-Aminoindol, 7-Hydroxyindol, 7-Ethyl-6-(β-hydroxyethyl)-aminoindol, 4-Aminoindol, 6-Hydroxy-1-methylindol, 5,6-Dihydroxyindol, 4-Hydroxy-1-N-methylindol, 4-Hydroxy-2-methylindol und 4-Hydroxy-5-methylindol sowie den Additionssalzen dieser Verbindungen mit einer Säure

**5.** Zusammensetzung nach Anspruch 1 oder 2, dadurch gekennzeichnet, daß die Benzimidazolderivate der Formel (III) ausgewählt sind unter 4-Hydroxybenzimidazol, 4-Aminobenzimidazol, 4-Hydroxy-7-methylbenzimidazol, 4-Hydroxy-2-methylbenzimidazol, 1-Butyl-4-hydroxybenzimidazol, 4-Amino-2-methylbenzimidazol, 5,6-Dihydroxybenzimidazol, 5-Hydroxy-6-methoxybenzimidazol, 4,7-Dihydroxybenzimidazol, 4,7-Dihydroxy-1-methylbenzimidazol, 4,7-Dimethoxybenzimidazol, 5,6-Dihydroxy-1-methylbenzimidazol, 5,6-Dihydroxy-2-methylbenzimidazol und 5,6-Dimethoxybenzimidazol sowie den Additionssalzen dieser Verbindungen mit einer Säure.

**6.** Zusammensetzung nach Anspruch 1 oder 2, dadurch gekennzeichnet, daß die Benzomorpholinderivate der Formel (IV) unter 6-Hydroxybenzomorpholin, N-Methyl-6-hydroxybenzomorpholin und 6-Aminobenzomporpholin sowie den Additionssalzen dieser Verbindungen mit einer Säure ausgewählt sind.

**7.** Zusammensetzung nach Anspruch 1 oder 2, dadurch gekennzeichnet, daß die Pyridinderivate der Formel (V) unter 2,6-Dihydroxy-4-methylpyridin, 2,6-Dihydroxy-3,4-dimethylpyridin, 2,6-Diaminopyridin und 3-Oxo-5-(3',5'-diamino-2'-pyridyloxy)-pentanol sowie den Additionssalzen dieser Verbindungen mit einer Säure ausgewählt sind.

8. Zusammensetzung nach Anspruch 1 oder 2, dadurch gekennzeichnet, daß die Indolinderivate der Formel (VI) unter 6-Hydroxyindolin, 6-Aminoindolin und 5,6-Dihydroxyindolin sowie den Additionssalzen dieser Verbindungen mit einer Säure ausgewählt sind.

9. Zusammensetzung nach Anspruch 1 oder 2, dadurch gekennzeichnet, daß die Chinolinderivate der Formel (VII) unter 6-Hydroxychinolin und 8-Amino-6-methoxychinolin sowie den Additionssalzen dieser Verbindungen mit einer Säure ausgewählt sind.

10. Zusammensetzung nach Anspruch 1 oder 2, dadurch gekennzeichnet, daß die Sesamolderivate der Formel (VIII) unter 2-Brom-4,5-methylendioxyphenol und 2-Methoxy-4,5-methylendioxyanilin sowie den Additionssalzen dieser Verbindungen mit einer Säure ausgewählt sind.

11. Zusammensetzung nach einem der Ansprüche 1 bis 10, dadurch gekennzeichnet, daß das oder die Diaminopyrazolderivate der Formel (I) 0,0005 bis 12 Gew.-% des Gesamtgewichts der Färbemittelzusammensetzung ausmachen.

12. Zusammensetzung nach Anspruch 11, dadurch gekennzeichnet, daß das oder die Diaminopyrazolderivate der Formel (I) 0,005 bis 6 Gew.-% des Gesamtgewichts der Färbemittelzusammensetzung ausmachen.

13. Zusammensetzung nach einem der Ansprüche 1 bis 12, dadurch gekennzeichnet, daß der oder die heterocyclischen Kuppler 0,0001 bis 10 Gew.-% des Gesamtgewichts der Färbemittelzusammensetzung ausmachen.

14. Zusammensetzung nach Anspruch 13, dadurch gekennzeichnet, daß der oder die heterocyclischen Kuppler 0,005 bis 5 Gew.-% des Gesamtgewichts der Färbemittelzusammensetzung ausmachen.

15. Zusammensetzung nach einem der Ansprüche 1 bis 14, dadurch gekennzeichnet, daß das zum Färben geeignete Medium (oder der Träger) aus Wasser oder einem Gemisch aus Wasser und mindestens einem organischen Lösungsmittel besteht, das unter den niederen $C_{1-4}$-Alkanolen, Glycerin, Glykolen und Glykolethern und aromatischen Alkoholen sowie deren Gemischen ausgewählt ist.

16. Zusammensetzung nach einem der Ansprüche 1 bis 15, dadurch gekennzeichnet, daß sie einen pH-Wert im Bereich von 3 bis 12 aufweist.

17. Verfahren zum Färben von Keratinfasern und insbesondere von menschlichen Keratinfasern, wie dem Haar, dadurch gekennzeichnet, daß auf die Fasern eine Färbemittelzusammensetzung nach einem der Ansprüche 1 bis 16 aufgetragen wird und daß die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

18. Verfahren nach Anspruch 17, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

19. Vorrichtung mit mehreren Abteilungen oder "Kit" zum Färben mit mehreren Abteilungen, wobei eine erste Abteilung eine Färbemittelzusammensetzung nach einem der Ansprüche 1 bis 16 und eine zweite Abteilung eine oxidierende Zusammensetzung enthält.